# FASCICULE DE BREVET EUROPEEN

(11) **EP 1 746 416 B1**
(45) Date de publication et mention de la délivrance du brevet: **30.06.2010**
(21) Numéro de dépôt: 06356096.5
(22) Date de dépôt: 21.07.2006
(51) Int. Cl.: G01N 33/34

(54) **Procédé de contrôle de la qualité alimentaire d'un papier et dispositif pour sa mise en oeuvre**
Verfahren und Vorrichtung zur Steuerung der Lebensmittelsqualität des Papiers
Method of controlling paper food grade and apparatus for carrying out said method

(30) Priorité: 21.07.2005 FR 0507771
(43) Date de publication de la demande: 24.01.2007
(73) Titulaire: Papeterie de Raon, 88110 Raon l'Etape (FR)
(72) Inventeur: Floccia, Louis, 38640 Claix (FR); Fernandes, Jean-Carlos, 38100 Grenoble (FR); Bortolotti, Marc, 88110 Raon L'Etape (FR); Gallaire, Pascal, 88600 Destord (FR); L'Huillier, Bénédicte, 88330 Moriville (FR)
(74) Mandataire: Maureau, Philippe

(56) Documents cités:
- EP-A- 0 559 305
- WO-A-97/46755
- US-A- 5 104 485
- US-A- 5 680 320

## Description

La présente invention a pour objet un procédé de contrôle de la qualité alimentaire d'un papier et un dispositif pour sa mise en oeuvre.

Il n'existe actuellement en Europe que quelques normes nationales concernant le contrôle alimentaire pour les papiers et cartons. Ces normes nationales imposent à une société fabriquant des papiers et cartons à usage alimentaire d'apporter la preuve, tous les deux ou trois ans que les produits fabriqués ne contiennent pas ou contiennent dans une certaine limite certains composés chimiques. Cette liste positive ou négative de composés chimiques n'est pas exhaustive et ne garantit pas au consommateur l'innocuité du papier utilisé. En effet, les certificats de conformité délivrés actuellement en Europe précise bien que l'aptitude n'est donnée que par rapport aux échantillons de papier analysés lors de la certification et ne tiennent pas compte des variations possibles de procédé ou de conditions de fabrication.

Une méthode connue pour mesurer la toxicité d'un papier consiste à utiliser une méthode d'analyse de cytotoxicité de l'eau de trempage d'une feuille de papier, voir Fauris et al., Cytotoxicological safety assessment of papers and boards used for food packaging, Food Additives and Contaminants, 1998, 15(6), pp 716 - 728, XP007906653. Il s'agit donc de déterminer si cette eau de trempage est propre à être consommée ou non, en s'aidant des méthodes de toxicologie employées pour déterminer si une eau est potable ou non. Toutefois, une telle analyse est longue à mettre en oeuvre, et peu compatible à un contrôle continu ou selon une périodicité importante, par exemple plusieurs fois par jour, sachant qu'un papier est produit en continu généralement 24h/24 et 365 jours par an. En effet, les analyses toxicologiques sont en pratique réalisées au mieux tous les quinze jours.

Le problème technique à la base de l'invention est de fournir un procédé de contrôle de la qualité alimentaire d'un papier, qui puisse être mis en oeuvre de façon quotidienne, ou plusieurs fois par jour, tout en possédant un excellent degré de fiabilité, analogue à celui fournit par une analyse de cytotoxicologie.

Le procédé selon l'invention, tel que défini par la revendication 1, vise à résoudre ce problème technique et est caractérisé en ce qu'il consiste, pour un type de papier déterminé :
- à effectuer une analyse de cytotoxicité d'un échantillon de référence de ce papier et/ou de la matière première de celui-ci puis, si la valeur mesurée est bonne selon les normes en vigueur,
- à effectuer une analyse du même échantillon par chromatographie en phase gazeuse en vue d'obtenir un spectre de référence, puis
- lors de la fabrication d'un papier conforme à l'échantillon de référence, à effectuer régulièrement des prélèvements d'échantillon de la matière première et/ou du papier sortant de fabrication,
- à effectuer une analyse de chaque échantillon par chromatographie en phase gazeuse,
- à comparer l'aire et la nature des pics du spectre de chaque échantillon à l'aire et à la nature des pics du spectre de l'échantillon de référence, puis
- si certains pics du spectre de l'échantillon analysé diffèrent de ceux de l'échantillon de référence, à analyser la nature des produits ayant généré ces pics, en vue de déterminer leur éventuelle toxicité.

Il a été découvert qu'existait une corrélation entre les notes de cytotoxicité et l'aire et la nature des pics de chromatographie en phase gazeuse. Il est donc possible d'établir pour un même échantillon une analyse de cytotoxicité et d'effectuer une chromatographie en phase gazeuse fournissant un spectre de référence. Dans la mesure où l'analyse de cytotoxicité est satisfaisante pour ce carton ou ce papier, la courbe chromatographique forme une courbe de référence à laquelle sont comparées des courbes obtenues par chromatographie en phase gazeuse d'échantillons prélevés régulièrement sur la chaîne de fabrication. S'il n'y a pas de distorsion entre les courbes des échantillons et la courbe de référence, il est possible de conclure que les échantillons présentent une bonne qualité alimentaire. Si au contraire, un chromatogramme montre des pics différents de ceux du chromatogramme de référence, il convient d'analyser la nature des produits ayant généré ces pics en vue de déterminer leur éventuelle toxicité.

Avantageusement, ce procédé consiste à réaliser l'analyse de cytotoxicité sur l'eau de trempage du papier ou de la matière première, sans dilution.

Les conditions d'analyse de cytotoxicité sont donc très strictes.

Suivant un mode de mise en oeuvre de ce procédé, celui-ci consiste à effectuer régulièrement une analyse de cytotoxicité d'un échantillon sortant de fabrication et une analyse par chromatographie en phase gazeuse de cet échantillon dont le spectre constituera le spectre de référence, jusqu'à la double mesure suivante.

Il est ainsi possible de procéder à une analyse de cytotoxicité par exemple toutes les deux semaines alors que les analyses d'échantillon par chromatographie en phase gazeuse seront effectuées plusieurs fois par jour.

La fréquence de réalisation des doubles analyses fournissant un spectre de référence pourrait être différente de deux semaines et correspondre à un nombre plus important de semaines.

Suivant un mode de mise en oeuvre, ce procédé consiste, dans le cas d'un échantillon dont le spectre obtenu par chromatographie en phase gazeuse, comporte un ou des pics différents de ceux du spectre de référence, et que ces pics ne sont pas attribués de façon certaine à des produits non toxiques, à effectuer une mesure de cytotoxicité.

Avantageusement, dans ce dernier cas, le procédé selon l'invention consiste à constituer une base de données contenant les spectres des échantillons de référence et des échantillons non conformes aux échantillons de référence, et à comparer à ces différents spectres un spectre non conforme au spectre de l'échantillon de référence pour la période en cours, en vue de déterminer si la qualité alimentaire de cet échantillon est bonne ou non.

La base de données est donc évolutive et actualisée.

Pour la mise en oeuvre de ce procédé, l'invention concerne également un dispositif tel que défini par la revendication 7.

Avantageusement, ce dispositif à microprocesseur contient en mémoire les spectres des échantillons conformes aux échantillons de référence et les spectres des échantillons non conformes aux échantillons de référence avec indication pour tous de la qualité alimentaire, bonne ou non acceptable.

L'invention est décrite ci-après en référence au dessin schématique annexé représentant un dispositif pour la mise en oeuvre du procédé selon l'invention ainsi que des chromatogrammes de plusieurs échantillons.
Figure 1 est une vue schématique d'un dispositif pour la mise en oeuvre du procédé,
Figures 2 à 7 sont des vues de différents chromatogrammes d'échantillons.

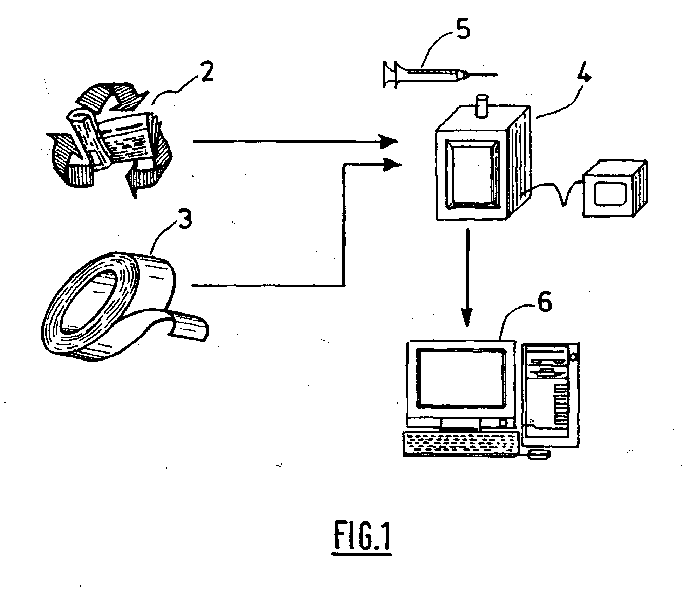

La figure 1 représente très schématiquement un dispositif pour la mise en oeuvre du procédé.

A la figure 1, la matière première utilisée pour la fabrication du papier est désignée par la référence 2, du papier sortant de fabrication désigné par la référence 3, un appareil de chromatographie en phase gazeuse est désigné par la référence 4, cet appareil étant éventuellement relié à un spectromètre de masse. Une seringue 5 indique que les échantillons de matière première ou de papier sont introduits pour analyse dans l'appareil de chromatographie. Le chromatogramme est fourni à un ordinateur 6 qui compare le chromatogramme à un chromatogramme de référence, cet ordinateur 6 assurant éventuellement, la gestion d'une base de données contenant des spectres des échantillons de référence, et des spectres d'échantillons non-conformes aux échantillons de référence. Il est ainsi possible pour l'ordinateur de déterminer si un spectre n'est pas conforme au spectre de référence et comporte des pics qui ont déjà été identifiés dans des spectres précédemment mémorisés, si ces pics correspondent à des substances dangereuses ou non et de conclure sur la toxicité ou non de l'échantillon analysé.

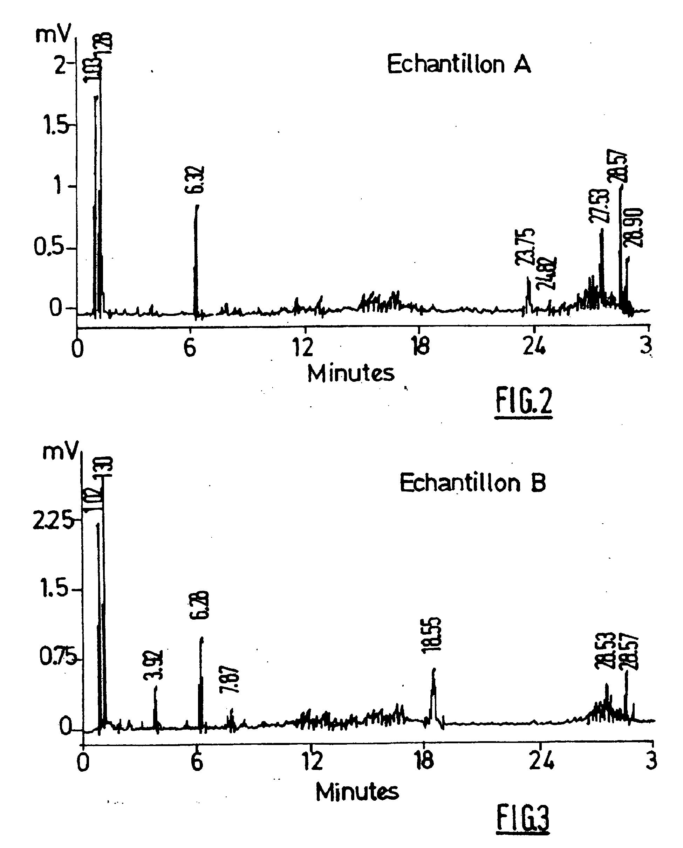

Les figures 2 et 3 représentent deux chromatogrammes correspondant à des échantillons A et B provenant de la fabrication de la même qualité de papier avec le même grammage.

La note de cytotoxicité pour le papier A est bonne car elle se situe au-dessus de la limite d'acceptabilité fixée par le laboratoire d'analyses des eaux qui est de 70%.

L'échantillon B fabriqué plus tard présente également une note de cytotoxicité supérieure à 70%. Le spectre de chromatographie de l'échantillon B est plus fourni mais peut-être accepté comme étant correct par la note de cytotoxicité. Il est donc possible de conclure qu'à ces limites de concentration (aire des pics), les pics en plus de l'échantillon B ayant un temps de rétention de : 3,92 minutes, 7,87 minutes et 18,55 minutes sont des pics correspondant à des substances inertes. Le spectre de l'échantillon B qui est plus complet que celui de l'échantillon A peut donc devenir une nouvelle référence mémorisée comme telle dans la base de données.

Comme indiqué précédemment, pour les tests de cytotoxicité c'est l'eau de trempage sans aucune dilution qui est mise en oeuvre. Il s'agit donc de conditions d'expérimentation très sévères.

Si la note de cytotoxicité est supérieure à 70%, la qualité alimentaire du papier est bonne. Si la note est comprise entre 50 et 70%, des tests complémentaires doivent être réalisés. Si la note est inférieure à 50%, le papier est considéré ne pas être apte au contact alimentaire.

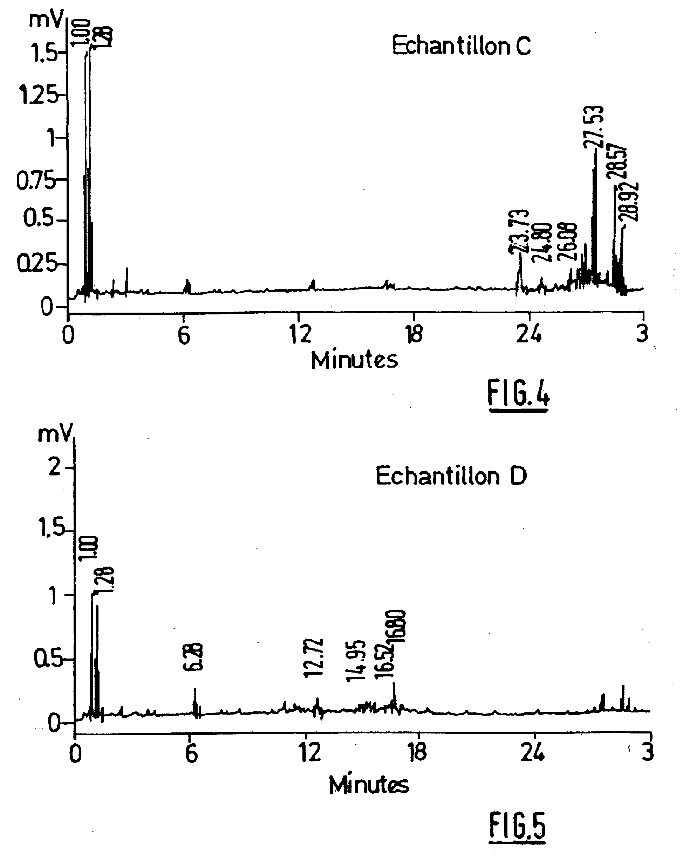

Un second exemple est donné en référence aux figures 4 et 5 concernant respectivement un échantillon C et un échantillon D.

L'échantillon C présente une note de cytotoxicité de 82,5% et l'échantillon D présentant une note de cytotoxicité de 53,5% est donc mis en attente de tests complémentaires.

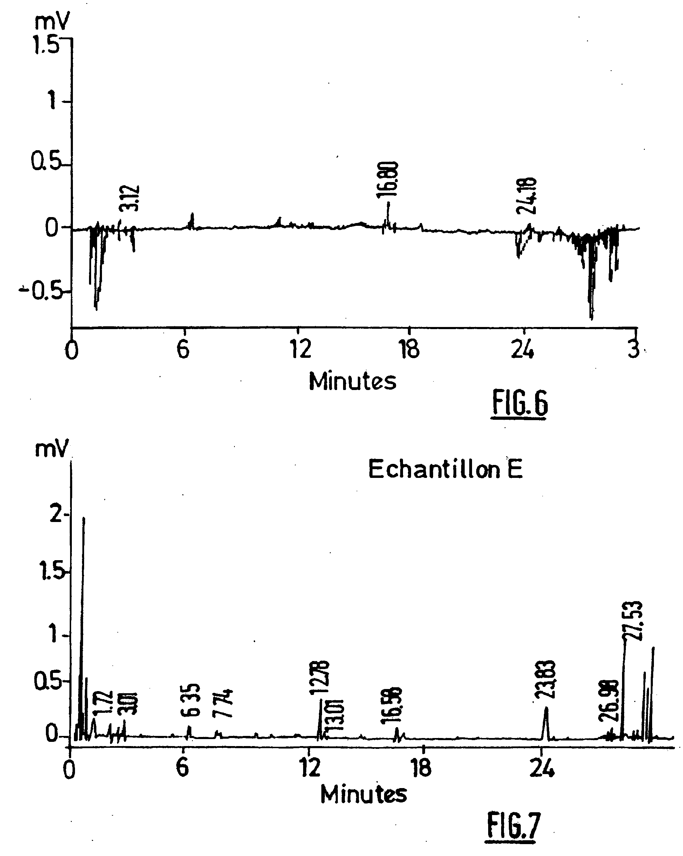

Afin de mieux comparer les spectres entre eux, il est possible de procéder à divers traitements des signaux issus du chromatographe ou du spectrophotomètre de masse. Il est possible soit de superposer deux courbes, soit de réaliser des courbes composites résultant de la soustraction d'un spectre par rapport à une référence. Tel est le cas pour la courbe de figure 6 qui résulte de la soustraction de la courbe échantillon C de la courbe échantillon D.

Dans ce cas, les pics apparaissant dans la zone positive sont des pics en plus ou ayant une aire supérieure, et les pics présents dans la zone négative sont des pics qui n'existent pas dans la courbe nouvelle ou en quantité plus importante dans la courbe de référence c'est-à-dire dans la courbe correspondant à l'échantillon C.

Il peut être constater dans la courbe de la figure 6 que deux pics ressortent dans la zone positive, dont un pic plus important en taille.

Le pic situé sensiblement à 6,30 minutes est un pic représentant vraisemblablement un dérivé de la dégradation de la cellulose comme de l'hexanal. Le pic situé approximativement à 16,80 minutes est un dérivé d'acide gras vraisemblablement issu de sous-produits d'agents de collage.

L'hexanal en tant que tel n'est pas reconnu comme un toxique pour l'homme à faible dose.

Le composé situé à 16,80 minutes n'a pas été étudié en terme de toxicologie.

Toutefois, cette méthode permet de démontrer que le niveau de contamination basé sur l'aire des pics peut affecter d'une manière négative la note de cytotoxicité.

II est donc utile, comme indiqué précédemment, de disposer d'une base de données qui soit la plus riche possible, afin de rapprocher le mieux possible les spectres obtenus par chromatographie des mesures de cytotoxicité, en vu d'obtenir un contrôle excellent de la toxicité globale.

L'échantillon de la figure 7 illustre un troisième exemple. Il s'agit d'un papier fabriqué à partir de fibres vierges, avec une note de toxicité anormalement inhabituelle de 1%. Ce papier est donc extrêmement toxique et contient peut-être un élément qui est extrêmement toxique.

Le spectre chromatographique de ce papier ne peut pas servir de référence, mais son analyse par la recherche des pics inhabituel permet d'identifier un pic particulier situé à environ 3 minutes. Ce pic peut être attribué à des composés de la famille des boranes qui sont des toxiques potentiels.

Comme il ressort de ce qui précède, le procédé selon l'invention apporte une grande amélioration à la technique existante, en permettant de procéder à un contrôle de la toxicité du papier alimentaire, en sortie de fabrication, à des fréquences très élevées, avec une excellente fiabilité.

## Revendications

1. Procédé de contrôle de la qualité alimentaire d'un papier, **caractérisé en ce qu'**il consiste, pour un type de papier déterminé :
- à effectuer une analyse de cytotoxicité d'un échantillon de référence de ce papier et/ou de la matière première de celui-ci puis, si la valeur mesurée est bonne selon les normes en vigueur,
- à effectuer une analyse du même échantillon par chromatographie en phase gazeuse en vue d'obtenir un spectre de référence, puis
- lors de la fabrication d'un papier (3) conforme à l'échantillon de référence, à effectuer régulièrement des prélèvements d'échantillon de la matière première et/ou du papier sortant de fabrication,
- à effectuer une analyse de chaque échantillon par chromatographie en phase gazeuse (4),
- à comparer l'aire et la nature des pics du spectre de chaque échantillon à l'aire et à la nature des pics du spectre de l'échantillon de référence (5), puis
- si certains pics du spectre de l'échantillon analysé diffèrent de ceux de l'échantillon de référence, à analyser la nature des produits ayant généré ces pics, en vue de déterminer leur éventuelle toxicité.

2. Procédé de contrôle selon la revendication 1, **caractérisé en ce qu'**il consiste à réaliser l'analyse de cytotoxicité sur l'eau de trempage du papier ou de la matière première, sans dilution.

3. Procédé de contrôle selon l'une des revendications 1 et 2, **caractérisé en ce qu'**il consiste à effectuer régulièrement une analyse de cytotoxicité d'un échantillon sortant de fabrication et une analyse par chromatographie en phase gazeuse de cet échantillon dont le spectre constituera le spectre de référence, jusqu'à la double mesure suivante.

4. Procédé de contrôle selon la revendication 3, **caractérisé en ce que** la fréquence de réalisation des doubles analyses fournissant un spectre de référence est de plusieurs semaines.

5. Procédé selon l'une des revendications 1 à 4, **caractérisé en ce qu'**il consiste, dans le cas d'un échantillon dont le spectre obtenu par chromatographie en phase gazeuse comporte un ou des pics différents de ceux du spectre de référence et que ces pics ne sont pas attribués de façon certaine à des produits non toxiques, à effectuer une mesure de cytotoxicité.

6. Procédé selon l'une des revendications 1 à 5, **caractérisé en ce qu'**il consiste à constituer une base de données contenant les spectres des échantillons de référence et des échantillons non conformes aux échantillons de référence, et à comparer à ces différents spectres un spectre non conforme au spectre de l'échantillon de référence pour la période en cours, en vue de déterminer si la qualité alimentaire de cet échantillon est bonne ou non.

7. Dispositif pour la mise en oeuvre du procédé selon l'une des revendications 1 à 6, **caractérisé en ce qu'**il comprend un appareil de chromatographie en phase gazeuse (4), couplé ou non à spectromètre de masse, relié à un dispositif à microprocesseur (6) mémorisant le spectre de référence d'un échantillon et comparant l'aire et la nature des pics du spectre de chaque nouvel échantillon à l'aire et à la nature des pics du-dit spectre de référence.

8. Dispositif selon la revendication 7 pour la mise en oeuvre du procédé selon la revendication 6, **caractérisé en ce que** le dispositif à microprocesseur (6) contient en mémoire les spectres des échantillons conformes aux échantillons de référence et les spectres des échantillons non conformes aux échantillons de référence avec indication pour tous de la qualité alimentaire, bonne ou non acceptable.

## Claims

1. A method for controlling the food grade of paper, **characterized in that** for a determined paper type it consists in:
- running a cytotoxicity assay of a reference sample of this paper and/or of the raw material thereof, and then, if the measured value is good according to valid standards,
- running an assay of the same sample by gas chromatography in order to obtain a reference spectrum, and then,
- when producing paper (3) according to the reference sample, regularly sampling the raw material and/or paper leaving the production stage,
- running an assay of each sample by gas chromatography (4),
- comparing the area and the nature of the spectrum peaks of each sample with the area and the nature of the spectrum peaks of the reference sample (5), and then,
- if certain spectrum peaks of the analyzed sample are different from those of the reference sample, analyzing the nature of the products having produced such peaks, so as to determine the possible toxicity thereof.

2. The control method according to claim 1, **characterized in that** it consists in running a cytotoxicity assay on the soaking water of the paper or raw material, without dilution.

3. The control method according to any of claims 1 and 2, **characterized in that** it consists in regularly running a cytotoxicity assay of a sample leaving the production stage and an analysis by gas chromatography of this sample, the spectrum of which will be the reference spectrum up to the next double measurement.

4. The control method according to claim 3, **characterized in that** the frequency for running double assays providing a reference spectrum is several weeks.

5. The method according to any of claims 1 to 4, **characterized in that** it consists in running a cytotoxicity measurement in the case of a sample, the spectrum of which, obtained by gas chromatography, comprises one or several peaks which are different from those of the reference spectrum, and where such peaks are not assigned with certainty to nontoxic products.

6. The method according to any of claims 1 to 5, **characterized in that** it consists in establishing a database containing the spectra of the reference samples and the samples not in line with the reference samples, and comparing with these different spectra a spectrum not in line with the spectrum of the reference sample for the current period of time, so as to determine whether the food grade of this sample is good or not.

7. A device for implementing the method according to any of claims 1 to 6, **characterized in that** it comprises a gas chromatography apparatus (4) coupled or not with a mass spectrometer, connected to a microprocessor device (6) storing the reference spectrum of a sample and comparing the area and nature of the spectrum peaks of each new sample with the area and nature of the peaks of said reference spectrum.

8. The device according to claim 7 for implementing the method according to claim 6, **characterized in that** the microprocessor device (6) holds in memory the spectra of the samples in line with the reference samples and the spectra of the samples not in line with the reference samples, while indicating for all of them the food grade, whether good or unacceptable.

## Patentansprüche

1. Verfahren zum Kontrollieren der Lebensmittelqualität von Papier, **dadurch gekennzeichnet, dass** es darin besteht für eine bestimmte Papierart:
- einen Cytotoxizitätstests einer Referenzprobe dieses Papiers und/oder seines Rohmaterials vorzunehmen, und dann, wenn der gemessene Wert gemäß den geltenden Normen korrekt ist,
- einen Test derselben Probe durch Gaschromatographie vorzunehmen, um ein Referenzspektrum zu erzielen, und dann,
- bei der Herstellung von Papier (3), das der Referenzprobe entspricht, regelmäßig Probenahmen des Rohmaterials und/oder des aus der Fertigung kommenden Papiers vorzunehmen,
- einen Test jeder Probe durch Gaschromatographie (4) vorzunehmen,
- die Fläche und die Beschaffenheit der Spektrumspitzen jeder Probe mit der Fläche und der Beschaffenheit der Spektrumspitzen der Referenzprobe (5) zu vergleichen, und dann,
- wenn bestimmte Spektrumspitzen der analysierten Probe anders als die der Referenzprobe sind, die Beschaffenheit der Produkte, die diese Spitzen hervorgebracht haben, zu analysieren, um ihre mögliche Toxizität zu bestimmen.

2. Kontrollverfahren nach Anspruch 1, **dadurch gekennzeichnet, dass** es darin besteht, den Cytotoxizitätstest am Einweichwasser des Papiers oder des Rohmaterials ohne Verdünnung auszuführen.

3. Kontrollverfahren nach einem der Ansprüche 1 und 2, **dadurch gekennzeichnet, dass** es darin besteht, regelmäßig einen Cytotoxizitätstest einer die Fertigung verlassenden Probe und eine Gaschromatographie-Bestimmung dieser Probe, deren Spektrum bis zur nächsten Doppelmessung das Referenzspektrum bildet, vorzunehmen.

4. Kontrollverfahren nach Anspruch 3, **dadurch gekennzeichnet, dass** die Ausführungsfrequenz von Doppelbestimmungen, die ein Referenzspektrum bereitstellen, mehrwöchig ist.

5. Verfahren nach einem der Ansprüche 1 bis 4, **dadurch gekennzeichnet, dass** es darin besteht, bei einer Probe, deren Spektrum, das durch Gaschromatographie erzielt wird, eine oder mehrere Spitzen umfasst, die anders als die des Referenzspektrums sind und diese Spitzen nicht mit Sicherheit ungiftigen Produkten zugeordnet werden können, eine Cytotoxizitätsmessung vorzunehmen.

6. Verfahren nach einem der Ansprüche 1 bis 5, **dadurch gekennzeichnet, dass** es darin besteht, eine Datenbank zu erstellen, welche die Spektren der Referenzproben und der Proben, die nicht den Referenzproben entsprechen, enthält, und mit diesen andersartigen Spektren ein Spektrum zu vergleichen, das nicht dem Spektrum der Referenzprobe für den derzeitigen Zeitraum entspricht, um zu bestimmen, ob die Lebensmittelqualität dieser Probe korrekt ist oder nicht.

7. Vorrichtung zum Durchführen des Verfahrens nach einem der Ansprüche 1 bis 6, **dadurch gekennzeichnet, dass** sie einen Gaschromatographen (4) umfasst, der mit einem Massenspektrometer gekoppelt ist oder nicht und an eine Mikroprozessor-Vorrichtung (6) angeschlossen ist, die das Referenzspektrum einer Probe speichert und die Fläche und Beschaffenheit der Spektrumspitzen jeder neuen Probe mit der Fläche und Beschaffenheit der Spitzen des Referenzspektrums vergleicht.

8. Vorrichtung nach Anspruch 7 zum Durchführen des Verfahrens nach Anspruch 6, **dadurch gekennzeichnet, dass** die Mikroprozessor-Vorrichtung (6) im Speicher die Spektren der Proben, die mit den Referenzproben übereinstimmen, und die Spektren der Proben, die nicht mit den Referenzproben übereinstimmen, speichert, wobei für alle davon die korrekte oder unannehmbare Lebensmittelqualität angegeben wird.
